# EUROPEAN PATENT APPLICATION

(11) **EP 4 487 813 A1**
(43) Date of publication of application: **08.01.2025**
(21) Application number: 24179227.4
(22) Date of filing: 31.05.2024
(51) Int. Cl.: A61D 9/00

(54) **KNEE JOINT ORTHOSIS**

(30) Priority: 05.07.2023 PL 44548823
(71) Applicant: WIMBA spolka z ograniczona odpowiedzialnoscia, 31-553 Krakow (PL)
(72) Inventor: KRUZLAK, Sylwia, 34-212 Bienkowka (PL); NOWAKOWSKI, Szymon, 30-147 Kraków (PL)
(74) Representative: Górska, Anna

(57) **Abstract**

A knee joint orthosis for animals comprising an upper support module (1) constituting an upper hoop (5) provided with two arms (6) connected by hinges (7) to a lower support module (2), which is provided with a middle hoop (3) and a lower hoop (4) as well as a replaceable token (25) placed in front of the lower support module (2), each of the hinges (7) being provided with an oval stop (10) provided with a recess (11).

## Description

The object of the invention is a knee joint orthosis for animals, in particular for dogs.

From American patent description no. US 9408738 B2 there are known orthopaedic braces and splints for use on a limb of an animal, that provide stability for unstable joints and limbs for short or long term treatment. They can be fitted immediately after corrective surgery, and can support and stabilise the joint while assisting or restricting the range of motion. The orthoses according to this invention are modular, thermoformable, and include an adjustable tensioning system.

From Korean patent description no. KR 102268890 B1 there is in turn known a knee orthosis for pets for rehabilitation after knee surgery comprising: a first supporting unit supported by the crus of a pet; and a second supporting unit supported by the calf; and an elastic joint mechanism detachably connected to both supporting units, allowing for adjustment of the angle of the animal's talocrural joint.

On the other hand, from international patent application no. WO 2022/112313 A1 there is known an orthosis adjusted for fitting on a joint of an animal, in particular a dog, comprising a first main element consisting of a hard shell and padding, and a second main element consisting of a hard shell and padding, the first and second main elements being connected by at least two sets of straps into a structure resembling a bivalve shell around the joint, thereby limiting the range of its flexion and extension movements.

The technical problem is the development of a new knee joint orthosis for animals, allowing for its adjustment to individually specified clinical needs of the animal, and capable of being modified during therapy.

The essence of the knee joint orthosis for animals according to the invention is in that an upper support module constitutes an upper hoop provided with two arms connected by hinges to a lower support module, which is provided with a middle hoop and a lower hoop as well as a replaceable token placed in front of the lower support module, each of the hinges being provided with an oval stop provided with a recess.

Preferably, the replaceable token is detachably connected to the lower support module.

Preferably, the replaceable token is placed in the central part of the middle hoop.

Preferably, the replaceable token has a shape resembling a prism, preferably a rectangular cuboid.

Preferably, the replaceable token is made of a flexible material, preferably polyurethane.

Preferably, the lower support module has the shape of a flat connector with a cross-section resembling the letter C, provided with the middle hoop on its upper end, and with the lower hoop on its lower end.

Preferably, the stop has a shape resembling a disc provided with a recess, the length of the recess corresponding to at least the width of the arm of the upper support module.

Preferably, the stop is provided on one surface thereof with an edge with a shape resembling a horseshoe, on which there are protrusions.

Preferably, the upper hoop, the middle hoop, and/or the lower hoop comprise a detachably connected fixed and moving part.

Preferably, the upper hoop, the middle hoop, and/or the lower hoop are provided with adjustable buckles.

Preferably, the upper hoop is provided with tunnels and a fixing string.

Preferably, the upper hoop, the middle hoop, and/or the lower hoop are provided with inserts.

Preferably, the lower hoop is provided with an insert provided with a recess whose shape corresponds to the outline of the Achilles tendon.

Preferably, the lower support module is connected to a talocrural joint module.

The knee joint orthosis for animals according to the invention is presented in an embodiment in the attached drawing, in which:

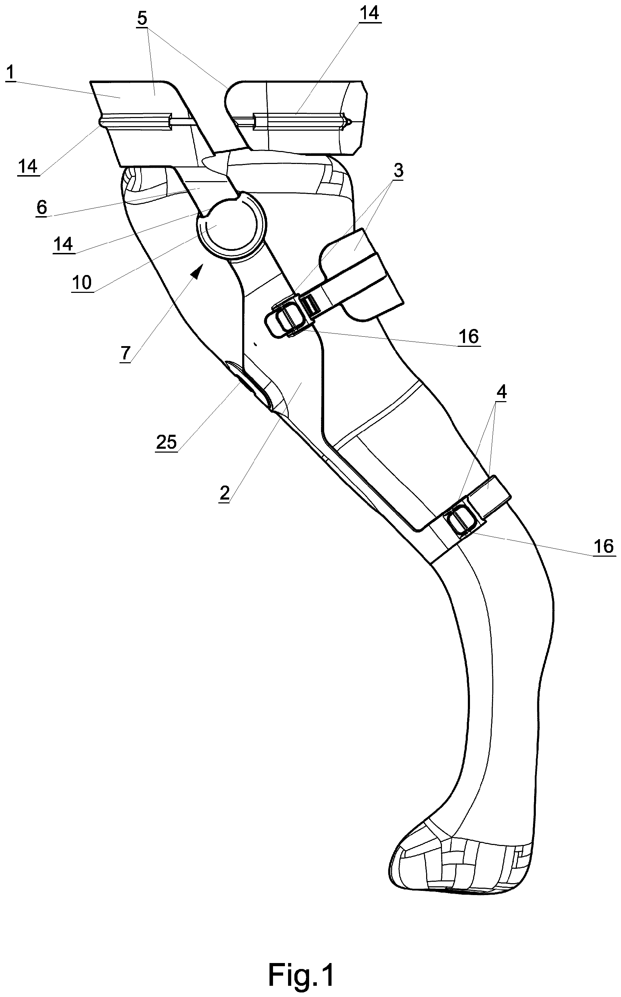

- Fig. 1: presents the orthosis in a general side view showing a part of an animal's limb;

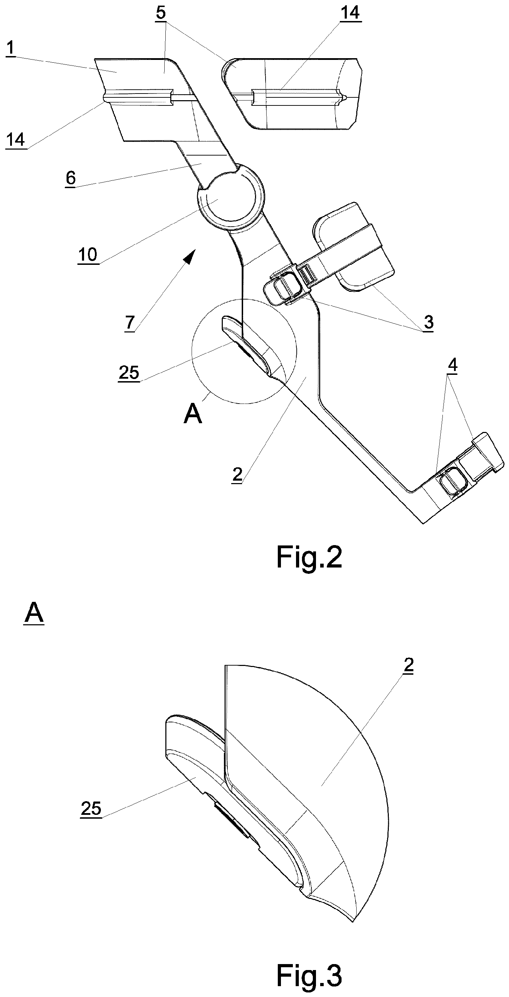

- Fig. 2: presents the orthosis in a general side view;
- Fig. 3: presents detail A of Fig. 2;

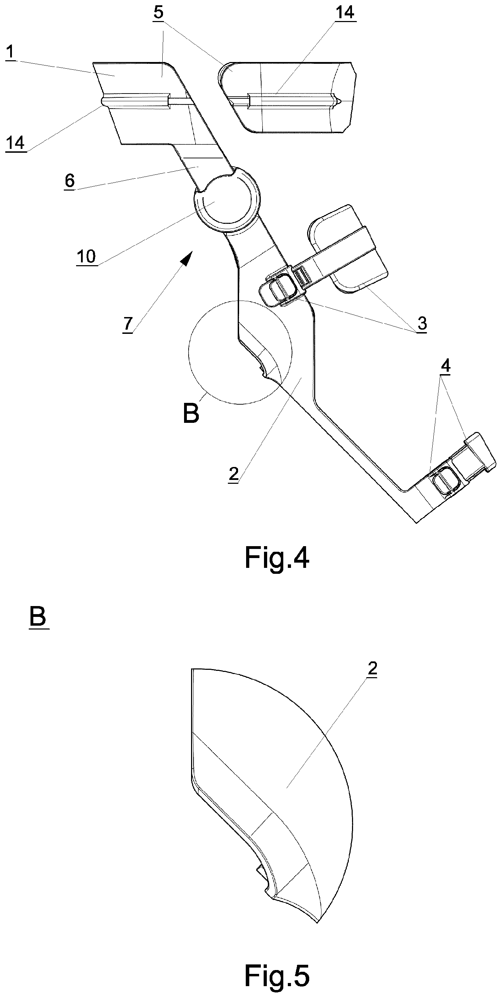

- Fig. 4: presents the orthosis in a general side view with the replaceable token **25** removed;
- Fig. 5: presents detail B of Fig. 4;

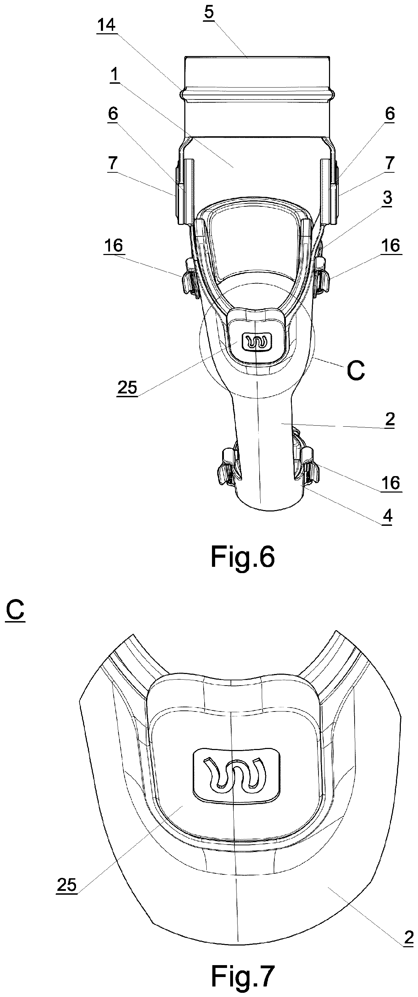

- Fig. 6: presents the orthosis in a perspective front view;
- Fig. 7: presents detail C of Fig. 6;

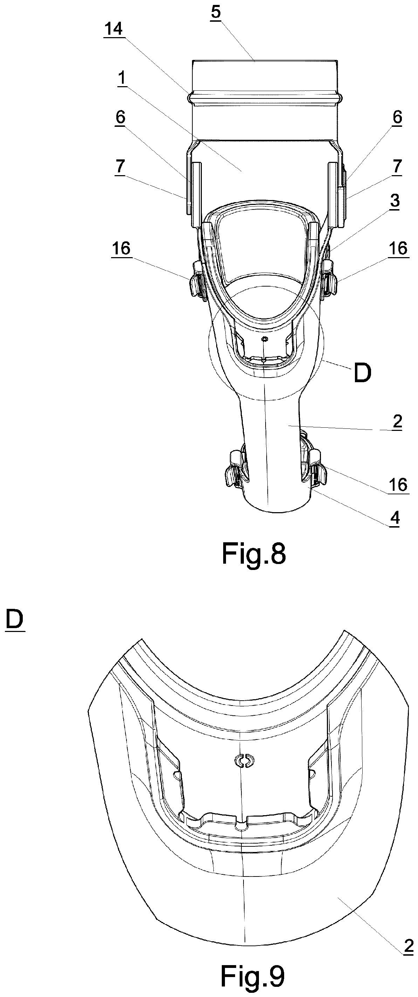

- Fig. 8: presents the orthosis in a perspective front view with the replaceable token **25** removed;
- Fig. 9: presents detail D of Fig. 8;

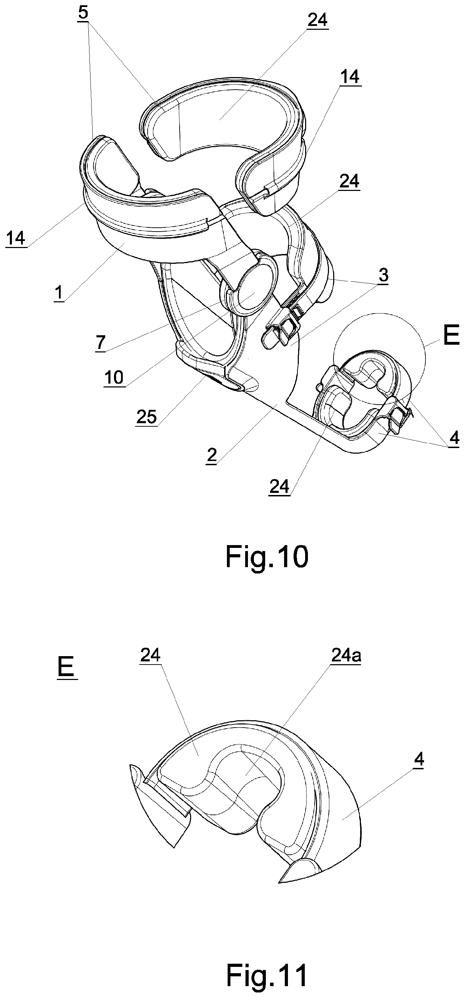

- Fig. 10: presents the orthosis in a perspective side view;
- Fig. 11: presents detail E of Fig. 9;

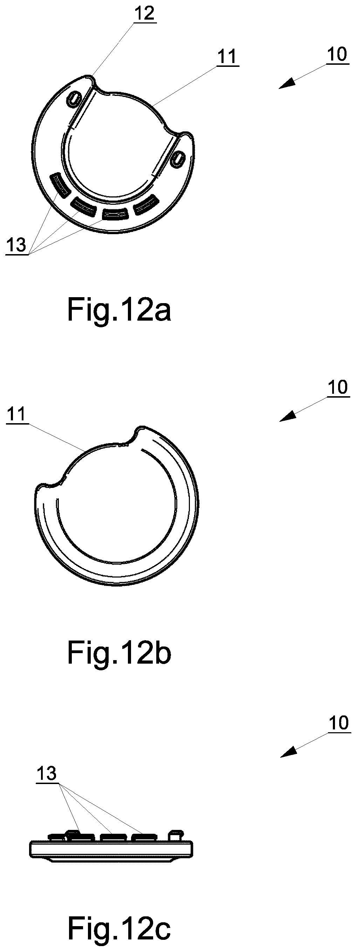

- Fig. 12a: presents the stop **10** in a general view from the inner side;
- Fig. 12b: presents the stop **10** in a general view from the outer side;
- Fig. 12c: presents the stop **10** in a general side view;

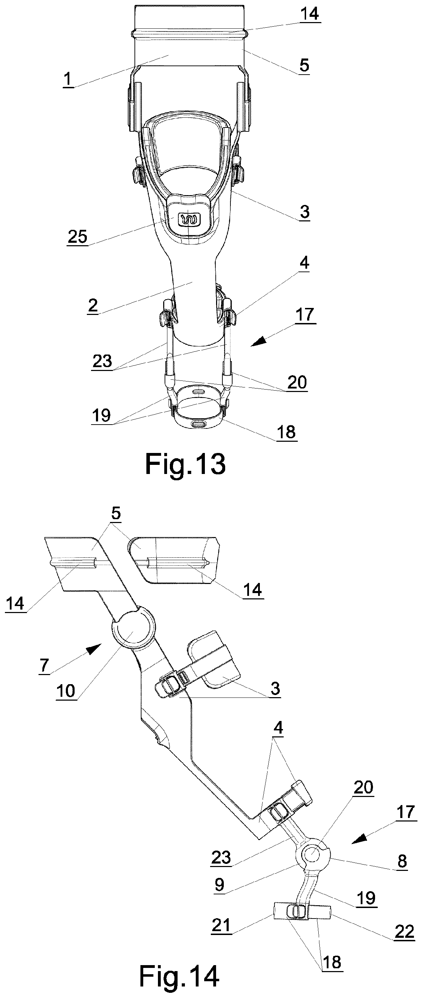

- Fig. 13: presents the orthosis in a perspective front view provided with a talocrural joint module **17;**
- Fig. 14: presents the orthosis in a general side view provided with a talocrural joint module **17;**

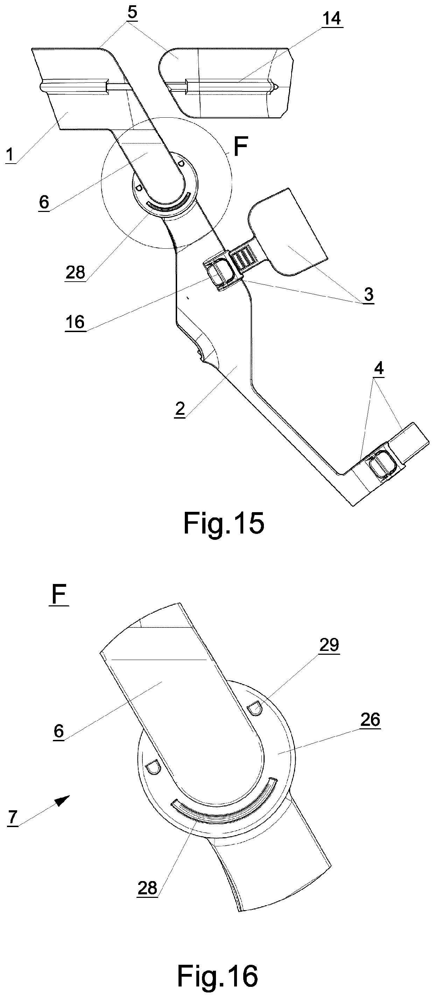

- Fig. 15: presents the orthosis in a general side view without the stop **10;**
- Fig. 16: presents detail F of Fig. 15;

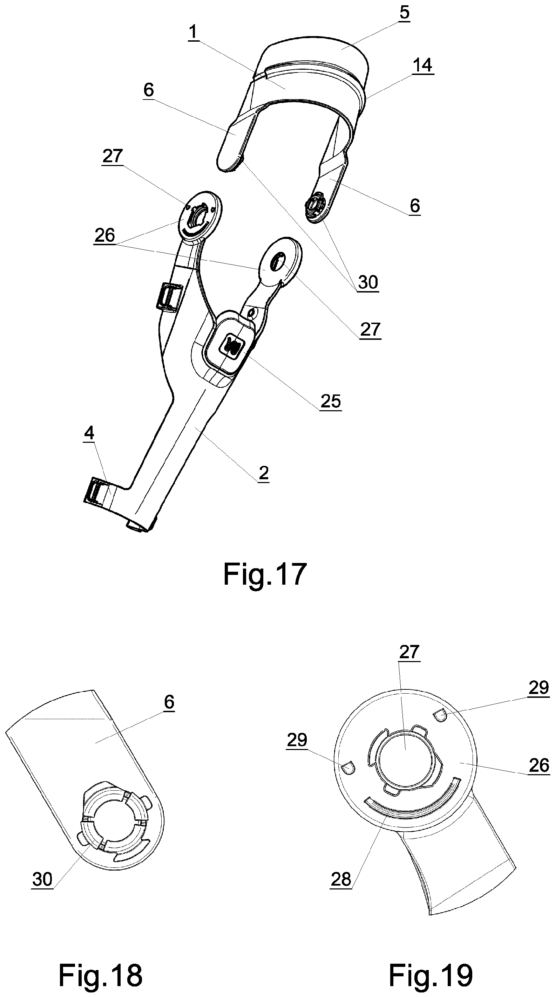

- Fig. 17: presents the orthosis in an exploded view;
- Fig. 18: presents a part of the arm **6** provided with a tongue **30** in a general front view;
- Fig. 19: presents the body **26** of the hinge **7** in a general front view.

The orthosis according to the invention and as presented in the drawing comprises an upper support module **1** and a lower support module **2.** The lower support module **2** has the shape of a flat connector with a cross-section resembling the letter C, provided with a middle hoop **3** on its upper end, and with a lower hoop **4** on its lower end. The upper support module **1** constitutes a hoop **5** provided with two arms **6** placed opposite each other. The arms **6** of the upper support module 1 and the lower support module **2** are connected by hinges **7.** Each of the hinges **7** is made of a circular body **26** permanently connected to the lower support module **2,** provided with a centrally placed opening **27** as well as an arcuate recess **28** placed parallel to the edge of the body, and mounting holes **29.** On the other hand, the inner surface of each of the arms **6** of the upper support module **1** is provided on its end with a circular tongue **30** with a shape corresponding to the opening **27** in the body **26** of the hinge **7.** The tongues **30** are locked in the openings **27** by means of recesses and protrusions. The axis of rotation of the hinge **7** is perpendicular to the axis of the orthosis. Each of the hinges **7** is provided with a stop **10.** The stop **10** preferably has a shape resembling a disc provided with a recess **11.** The length of the recess **11** corresponds to at least the width of the arm **6** of the upper support module **1.** The stop **10** is provided on one surface thereof with an edge **12** with a shape resembling a horseshoe. The edge **12** is provided with protrusions **13** used to mount the stop **10** on the hinge **7** or limit the angle of rotation of the hinge. The stop **10** allows for adjustment of the bending angle of the limb placed in the orthosis according to the invention within physiological ranges of movement, preferably from 42 to 162 degrees, and for blocking it, preferably in a position of 120 degrees.

Each of the hoops **3, 4, 5** comprises a fixed and a moving part, with them being detachably connected so as to enable opening and closing of the hoops **3, 4, 5.** The upper hoop **5** is provided on its outer surface with tunnels **14** used to place a fixing string therein-not shown. The upper hoop **5** is provided with an adjustable buckle (preferably with strings, straps or others), enabling the achievement of proper mobility in multiple axes, and enabling precise placement of the point of support for the orthosis on the animal's limb. Such a buckle provides stability of the orthosis on the limb, at the same time providing freedom of movement in the intended areas when the muscles of the limb work. The middle **3** and lower hoop **4** are provided with latching buckles **16,** with straps with tooth-based adjustment.

The length of the lower support module **2** is adjusted individually to the length of the limb of a specific animal, so that the middle hoop **3** is placed under the knee joint, with the lower hoop **4** above the talocrural joint near the Achilles tendon. In order to properly place the lower hoop **4** on the lower limb of an animal, the lower hoop **4** is provided with an insert **24,** provided with a recess **24a** whose shape corresponds to the outline of the Achilles tendon.

The lower support module **2** may be additionally connected to a talocrural joint module **17,** which comprises a talocrural joint hoop **18,** lateral straps **19,** and lower hinges **20.** The talocrural joint hoop **18** has a fixed **21** and a moving part **22,** the fixed part **21** and the moving part **22** being detachably connected, preferably by a latching connection, so as to enable opening and closing of the talocrural joint hoop **18.** In another embodiment, the fixed **21** and moving part **22** are detachably connected from one side only, and they are provided only with a single closure. Two lateral arms **19** are connected to the fixed part **21** of the talocrural joint hoop **18.** The lower part of each of the arms **19** is connected to the fixed part **21** of the talocrural joint hoop **18,** and the upper part of each of the arms **19** is connected to the lower hinge **20.** The lower hinges **20** comprise a sleeve **8** and a hoop **9,** the hoop **9** being placed inside the sleeve **8.** Arms **23** provided with a port-not shown-used to connect the arms **23** to the lower support module **2** are connected to the lower hinges **20.**

The inner surfaces of the upper hoop **5,** the middle hoop **3,** the lower hoop **4,** and the talocrural joint hoop **18** are provided with inserts **24,** preferably soft, printed from a flexible material, e.g. TPU. The inserts **24** allow for putting the orthosis stably on an animal's limb, increase the comfort of usage, and enable a precise fit of the orthosis on the limb. The insert **24** lining the interior of the lower hoop **4** has a shape adjusted to the shape of the animal's limb, enabling the orthosis to be supported on the tibia and, to a minor degree, on the fibula, at the same time relieving the Achilles tendon.

The lower support module **2** is provided with a replaceable token **25,** which is detachably connected thereto. The replaceable token **25** is placed in front of the lower support module **2,** in the central part of the middle hoop **4.** Preferably, the replaceable token **25** has a shape resembling a rectangular cuboid, and it is made of a flexible material, e.g. polyurethane. Preferably, the replaceable token **25** is a replaceable element with various dimensions, thus enabling additional adjustment of the point of support of the orthosis on the animal's tibia. The replaceable token **25** may rest against the tuberosity of the tibia, below or above it, depending on the type of surgical procedure and the surgeon's suggestion. It can also constitute a separate element of therapy, in which, through the use of sequential replaceable tokens **25** made in various shapes or of various materials, an increasing or decreasing pressure applied to the places of wearing is achieved within the knee joint.

The orthosis according to the invention is intended for treatment or rehabilitation of numerous diseases of the knee joint among animals. The manufacturing of the orthosis is based on the 3D scanning and 3D printing technology, thereby enabling precise preparation of the product on the basis of an animal's individual dimensions. The ability to replace the stops **10** and the replaceable tokens **25** allows for further individual adjustment of the orthosis to specific needs of therapy or rehabilitation. In addition, the knee joint orthosis can be connected to a talocrural joint module **17** in order to increase stability of the limb. The knee joint orthosis according to the invention is intended in particular for nonoperative supporting of stabilisation of the knee joint, postoperative controlled activation of a limb, as well for supporting the healing of soft tissues. The orthosis is found useful in healing various diseases, such as: cranial cruciate ligament rupture (CrCLR), medial patella luxation (MPL), lateral patella luxation (LPL), and osteoarthritis (OA).

## Claims

1. A knee joint orthosis for animals comprising an upper support module and a lower support module connected by hinges, **characterised in that** the upper support module (1) constitutes an upper hoop (5) provided with two arms (6) connected by the hinges (7) to the lower support module (2), which is provided with a middle hoop (3) and a lower hoop (4) as well as a replaceable token (25) placed in front of the lower support module (2), each of the hinges (7) being provided with an oval stop (10) provided with a recess (11).

2. The knee joint orthosis according to claim 1, **characterised in that** the replaceable token (25) is detachably connected to the lower support module (2).

3. The knee joint orthosis according to claim 1, **characterised in that** the replaceable token (25) is placed in the central part of the middle hoop (3).

4. The knee joint orthosis according to claim 1, **characterised in that** the replaceable token (25) has a shape resembling a prism, preferably resembling a rectangular cuboid.

5. The knee joint orthosis according to claim 1, **characterised in that** the replaceable token (25) is made of a flexible material, preferably polyurethane.

6. The knee joint orthosis according to claim 1, **characterised in that** the lower support module (2) has the shape of a flat connector with a cross-section resembling the letter C, provided with the middle hoop (3) on its upper end, and with the lower hoop (4) on its lower end.

7. The knee joint orthosis according to claim 1, **characterised in that** the stop (10) has a shape resembling a disc provided with a recess (11), the length of the recess (11) corresponding to at least the width of the arm (6) of the upper support module (1).

8. The knee joint orthosis according to claim 1, **characterised in that** the stop (10) is provided on one surface thereof with an edge (12) with a shape resembling a horseshoe, on which there are protrusions (13).

9. The knee joint orthosis according to claim 1, **characterised in that** the upper hoop (5), the middle hoop (3), and/or the lower hoop (4) comprise a detachably connected fixed and moving part.

10. The knee joint orthosis according to claim 1, **characterised in that** the upper hoop (5), the middle hoop (3), and/or the lower hoop (4) are provided with adjustable buckles.

11. The knee joint orthosis according to claim 1, **characterised in that** the upper hoop (5) is provided with tunnels (14) and a fixing string (15).

12. The knee joint orthosis according to claim 1, **characterised in that** the upper hoop (5), the middle hoop (3), and/or the lower hoop (4) are provided with inserts (24).

13. The knee joint orthosis according to claim 1, **characterised in that** the lower hoop (4) is provided with an insert (24) provided with a recess (24a) whose shape corresponds to the outline of the Achilles tendon.

14. The knee joint orthosis according to claim 1, **characterised in that** the lower support module (2) is connected to a talocrural joint module (17).
